# EUROPÄISCHE PATENTANMELDUNG

(11) **EP 4 652 914 A2**
(43) Veröffentlichungstag der Anmeldung: **26.11.2025**
(21) Anmeldenummer: 25170094.4
(22) Anmeldetag: 11.04.2025
(51) Int. Cl.: A61B 1/00, A61B 1/005, A61B 1/12, A61B 1/307

(54) **MEDIZINISCHES INSTRUMENT UND DESSEN HERSTELLUNG**

(30) Priorität: 21.05.2024 DE 102024114070
(71) Anmelder: Karl Storz SE & Co. KG, 78532 Tuttlingen (DE)
(72) Erfinder: KÄCHELE, Marvin, 78532 Tuttlingen (DE); HERMLE, Rainer, 78532 Tuttlingen (DE); KARL, Wolfgang, 78532 Tuttlingen (DE)

(57) **Zusammenfassung**

Ein medizinisches Instrument (30, 230) weist einen Schaft (34) auf, der sich zwischen einem distalen Ende (38) und einem proximalen Ende (40) erstreckt. Beim proximalen Ende (40) des Schafts (34) ist ein Gehäuse (36) vorgesehen. Beim distalen Ende (38) des Schafts (34) ist zumindest einen Effektor (60) vorgesehen. Das Instrument (30, 230) weist zumindest ein Steuerelement (72) zur mechanischen Steuerung des Effektors (60) auf. Das Steuerelement (72) erstreckt sich zumindest abschnittsweise durch ein sich parallel zum Schaft (34) erstreckendes Führungsrohr (70). Das Führungsrohr (70) ist zumindest abschnittsweise entlang seiner Längserstreckung (104) mit seitlichen Aussparungen (100) versehen, durch die das Führungsrohr (70) für Reinigungsmedien zugänglich ist. Ein Verfahren dient zur Herstellung eines Führungsrohres (70) eines medizinischen Instruments (30, 230).

## Beschreibung

Die vorliegende Offenbarung bezieht sich auf ein medizinisches Instrument, insbesondere auf ein Instrument in Form eines Arbeitseinsatzes für ein medizinisches System. Medizinische Systeme in Form mehrteiliger Instrumente sind bekannt, beispielhaft handelt es sich um Zystoskope (vergleiche auch Cysto-Urethroskope), Hysteroskope, Resektoskope oder dergleichen. Allgemein bezieht sich die vorliegende Offenbarung auf medizinische Instrumente für diagnostische, therapeutische und/oder chirurgische Maßnahmen in einem Hohlraum im Körper eines Patienten.

Zystoskope können grundsätzlich als flexible Zystoskope oder als starre Zystoskope gestaltet sein. Je nach konkretem Anwendungsfall können bei mehrteiligen Instrumenten verschiedene (Einzel-) Instrument miteinander kombiniert werden. Beispielhaft umfasst ein zystoskopisches System einen starren Außenschaft (Zystoskopschaft), der dazu ausgebildet ist, einen Einsatz aufzunehmen, der als Arbeitseinsatz und/oder Beobachtungseinsatz dient. Der Einsatz kann wiederum zur Aufnahme eines Beobachtungsinstruments (Endoskop) und/oder zur Aufnahme sonstiger Instrumente (flexible oder starre Instrumente wie Zangen, Greifer, Scheren, elektrochirurgische Instrumente und Ähnliches) dienen.

Ferner sind sogenannte Arbeitseinsätze bekannt, die die eine Auslenkung flexibler Instrumente bei deren distalen Ende erlauben. Zu diesem Zweck dient beispielhaft ein sogenannter Albarran-Hebel, der über Steuerelemente (Steuerkabel oder dergleichen) mechanisch mit einer Handhabe (Rad, Hebel oder dergleichen) bei einem proximalen Gehäuse gekoppelt ist. Auf diese Weise kann der Albarran-Hebel betätigt werden, um beim distalen Ende ein Instrument seitlich gegenüber der (globalen) Längserstreckung des Arbeitseinsatzes auszulenken. Mit anderen Worten kann ein Albarran-Hebel zur Steuerung der Orientierung/Auslenkung eines Katheters an einer Spitze eines Zystoskops dienen. Albarran-Hebel und ähnliche Mechanismen können auch bei anderen Instrumenten als Zystoskopen genutzt werden.

Ein mehrteiliges urologisches Instrument mit einem Albarran-Hebel ist beispielsweise aus der US 4,178,920 A bekannt. Zystoskope mit Albarran-Hebel sind ferner aus der DE 1 997 000 U und der DE 77 06 935 U1 bekannt. Aus der DE 100 09 020 A1 ist ein Instrument in Form eines Hysteroskops mit Albarran-Hebel bekannt. Ausgestaltungen von Albarran-Einsätzen sind ferner aus der EP 4 205 629 A1 und der EP 4 238 475 A1 bekannt.

Die Reinigung und Sterilisation von Instrumenten mit Albarran-Hebel und ähnlichen Instrumenten ist zuweilen herausfordernd, weil regelmäßig eine miniaturisierte Mechanik mit einer Führung des Steuerelements von proximal nach distal hin zum auslenkbaren Hebel gegeben ist. Die Führung bzw. das Steuerelement können verschmutzt/kontaminiert werden. Aufgrund der Miniaturisierung muss besondere Aufmerksamkeit darauf gelenkt werden, dass die verwendeten Reinigungsfluide auch tatsächlich die potenziell verunreinigten Bereiche erreichen.

Vermeintlich naheliegende Maßnahmen zur Erleichterung der Reinigung solcher Instrumente können häufig schon deshalb nicht umgesetzt werden, weil aufgrund der Miniaturisierung schlichtweg kein Bauraum vorhanden ist. Auch darf die Betätigung etwaiger Effektoren über Steuerelemente nicht beeinträchtigt werden, beispielsweise mit dem Blick auf die Positioniergenauigkeit und die Wiederholgenauigkeit. Die Reinigung/Sterilisation kann beispielsweise mit Dampf und/oder Reinigungsflüssigkeit erfolgen.

Es gibt Vorschriften und Hilfsmittel für die Reinigung von Albarran-Arbeitseinsätzen und ähnlichen Instrumenten. Es hat sich jedoch gezeigt, dass der Reinigungserfolg auch von der Erfahrung und dem Engagement der mit der Reinigung beauftragten Fachperson abhängt. Ferner ist zu beachten, dass die Reinigung der Instrumente teilweise hochgradig automatisiert und teilweise händisch erfolgt. In jedem Fall sollte das gewünschte Reinigungsergebnis sicher und reproduzierbar erreicht werden.

Vor diesem Hintergrund liegt der vorliegenden Offenbarung die Aufgabe zugrunde, ein medizinisches Instrument anzugeben, dass hinsichtlich der Reinigung und Sterilisation optimiert ist. Insbesondere sollen neuralgische Elemente und Abschnitte des Instruments gut für Reinigungsfluide erreichbar sein. Die verbesserte Reinigbarkeit soll möglichst ohne nachteilige Auswirkungen auf die Funktionalität des Instruments erzielbar sein. Insbesondere soll das Instrument als Albarran-Arbeitseinsatz zur Verwendung bei Zystoskopen und ähnlichen mehrteiligen Instrumenten nutzbar sein. Insbesondere soll die Führung für Steuerelemente eines Albarran-Hebels oder ähnlicher Effektoren hinsichtlich der Reinigbarkeit optimiert werden. Schließlich soll im Rahmen der vorliegenden Offenbarung ein Verfahren zur Herstellung eines Führungsrohres für ein medizinisches Instrument angegeben werden, wobei sich das Führungsrohr insbesondere für Steuerelemente für Albarran-Hebel und ähnliche Effektoren eignen und daher möglichst durchmesserklein gestaltet sein soll.

Gemäß einem ersten Aspekt bezieht sich die vorliegende Offenbarung auf ein medizinisches Instrument, insbesondere einen Arbeitseinsatz für ein Zystoskop, Hysteroskop oder Resektoskop, das Folgendes aufweist:
- einen Schaft, der sich zwischen einem distalen Ende und einem proximalen Ende erstreckt,
- ein Gehäuse beim proximalen Ende des Schafts,
- zumindest einen Effektor beim distalen Ende des Schafts,
- zumindest ein Steuerelement zur mechanischen Steuerung des Effektors,
   wobei sich das Steuerelement zumindest abschnittsweise durch ein sich parallel zum Schaft erstreckendes Führungsrohr erstreckt, und
   wobei das Führungsrohr zumindest abschnittsweise entlang seiner Längserstreckung mit seitlichen Aussparungen versehen ist, durch die das Führungsrohr für Reinigungsmedien zugänglich ist.

Die Aufgabe der Offenbarung wird auf diese Weise gelöst.

Ein offenbarungsgemäß gestaltetes Instrument weist eine verbesserte Reinigbarkeit auf, weil die Aussparungen des Führungsrohres ein Einströmen und Ausströmen von Reinigungsmedien ermöglichen. Damit kann ein potenziell problematischer Bereich zuverlässig gereinigt und sterilisiert werden.

Insbesondere handelt es sich bei dem Führungsrohr um ein durchmesserkleines Führungsrohr. Ein üblicher Durchmesser eines Schafts eines medizinischen Instruments beträgt beispielsweise 4 mm, 8 mm bis hin zu 10 mm oder gar 12 mm. Ein offenbarungsgemäßes Führungsrohr weist regelmäßig einen deutlich kleineren Durchmesser auf, beispielsweise einen Durchmesser von 2 mm oder 1 mm.

Der Schaft des Instruments kann einen Arbeitskanal bilden. Das zumindest eine Führungsrohr dient als Führung für ein Steuerelement zur Steuerung des Effektors. Der Effektor kann beispielhaft Bestandteil eines Auslenkmechanismus sein, etwa ein Auslenkhebel, insbesondere ein sogenannter Albarran-Hebel.

Das Steuerelement kann auch als Steuerkabel bezeichnet werden. Es kann sich dabei beispielhaft um einen Bowdenzug, einen Draht, eine Zug-Druck-Stange und ein ähnliches strangförmiges Steuerelement handeln. Es sind Steuerelemente bekannt, die primär auf Zug betätigt werden, so dass beispielsweise zwei Zustände des Effektors mit zwei Steuerelementen betätigt werden. Es sind jedoch auch Steuerelemente bekannt, die sowohl auf Zug als auch auf Druck betätigt werden.

Im Rahmen der vorliegenden Offenbarung handelt es sich bei einem proximalen Abschnitt oder Bereich um einen Abschnitt oder Bereich, der näher beim Beobachter/Anwender und weiter weg vom Sichtfeld/Patienten angeordnet ist als ein distaler Abschnitt oder Bereich. Gleichermaßen handelt es sich bei einem distalen Abschnitt oder Bereich um einen Abschnitt oder Bereich, der näher beim Sichtfeld/Patienten und weiter weg vom Beobachter/Anwender angeordnet ist als ein proximaler Abschnitt oder Bereich. Demgemäß kann distal auch als patientennah, dem Patienten zugewandt und/oder beobachterfern beschrieben werden. Proximal kann auch als patientenfern, dem Patienten abgewandt und/oder beobachternah beschrieben werden. Bei der Anwendung als endoskopisches Instrument ist üblicherweise das distale Ende des Schafts in den Körper eingeführt, um dort Beobachtungen vornehmen zu können. Zumindest das proximale Ende des Instruments ragt aus dem Körper heraus, weil dort die Handhabung und Steuerung durch den Bediener erfolgt.

Das distale Ende ist regelmäßig der distalen Endbereich des Schafts, also nicht nur eine äußerste Spitze davon. Das Instrument ist zumindest in beispielhaften Ausgestaltungen als starres Instrument mit einem starren Schaft gestaltet. Der Schaft definiert üblicherweise eine Haupterstreckungsrichtung des Instruments. Der Schaft des Instruments weist regelmäßig eine Längserstreckung auf, die ein Mehrfaches bis Vielfaches der Querstreckung (Durchmesser oder dergleichen) umfasst.

In beispielhaften Ausgestaltungen ist das zumindest eine Führungsrohr an einer Außenseite des Schafts angeordnet. Mit anderen Worten ist gemäß diesen Ausgestaltungen das Führungsrohr nicht innerhalb des Schafts positioniert.

Das Führungsrohr (und auch der Schaft) besteht üblicherweise aus Metallwerkstoffen, beispielsweise aus Edelstahl, insbesondere chirurgischem Edelstahl. Auch eine Herstellung aus anderen Metallen, beispielsweise aus einer Titanlegierung oder einer Aluminiumlegierung ist grundsätzlich vorstellbar.

Bei dem Instrument handelt es sich beispielhaft um einen Arbeitseinsatz für ein Zystoskop. Allgemein handelt es sich bei dem Instrument um ein Instrument für diagnostische und therapeutische Anwendungen, Untersuchung und Behandlung von Organen und Gewebe.

Die seitlichen Aussparungen können als Längsschlitze entlang der Längserstreckung des Führungsrohres bezeichnet werden. Durch die seitlichen Aussparungen besteht eine fluidische Verbindung zwischen dem Inneren des Führungsrohres und der Umgebung. Auf diese Weise können (flüssige oder gasförmige) Reinigungsfluide Einströmen und Ausströmen.

Das Gehäuse kann mehrteilig gestaltet sein. Der Schaft ist bei seinem proximalen Ende mit dem Gehäuse gekoppelt. Beispielhaft mündet der Schaft mit seinem proximalen Ende in das Gehäuse. Das Gehäuse erlaubt die Handhabung des Instruments. Ferner können Betätigungselemente und dergleichen beim Gehäuse angeordnet sein.

Gemäß einer beispielhaften Ausgestaltung ist der Effektor ein auslenkbarer Hebel am distalen Ende des Schafts, insbesondere ein Albarran-Hebel. Mit einem Albarran-Hebel können Kanülen und andere Instrumente beim distalen Ende des Schafts ausgelenkt werden. Der Grad der Auslenkung ist feinfühlig steuerbar. Steuerelemente für den Albarran-Hebel erstrecken sich beispielhaft durch offenbarungsgemäße Führungsrohre parallel zum Schaft zwischen dem Gehäuse und dem Albarran-Hebel. Komponenten der Steuerung des Albarran-Hebels sollen möglichst miniaturisiert sein, damit das medizinische System (beispielsweise Zystoskop) insgesamt bei einem gegebenen Außendurchmesser möglichst viele Funktionen bereitstellen kann.

Gemäß einer weiteren beispielhaften Ausgestaltung ist am Gehäuse ein Betätigungselement zur Betätigung des Effektors ausgebildet, mit dem das Steuerelement nach distal oder proximal bewegbar ist. Mit anderen Worten ist also das Betätigungselement über eine beim Gehäuse oder im Gehäuse angeordnete Effektorsteuerung sowie das zumindest eine Steuerelement mit dem Effektor verbunden. Das Betätigungselement ist beispielhaft ein Rad oder Hebel, das/der am Gehäuse gelagert ist.

Gemäß einer weiteren beispielhaften Ausgestaltung ist das Führungsrohr in einem ersten Umfangsabschnitt am Schaft befestigt, wobei die Aussparungen auf einem davon abgewandten zweiten Umfangsabschnitt des Führungsrohres ausgebildet sind. Auf diese Weise kann das Reinigungsfluid die Aussparungen gut passieren, ohne dass Beeinträchtigungen durch benachbarte Wandabschnitte des Schafts drohen. Gemäß einer beispielhaften Ausgestaltung ist das Führungsrohr außen am Schaft befestigt.

Gemäß einer weiteren beispielhaften Ausgestaltung bilden mehrere Aussparungen eine Reihe entlang der Längserstreckung des Führungsrohres. Beispielhaft handelt es sich um eine (einzige) Reihe von Längsschlitzen entlang der Längserstreckung des Führungsrohres. Es ist grundsätzlich auch vorstellbar, mehrere Reihen von Längsschlitzen in das Führungsrohr einzubringen. Die in einer Reihe angeordneten Aussparungen sind hintereinander angeordnet und jeweils voneinander beabstandet. Die Hauptstreckungsrichtung der Reihe ist parallel zur Längserstreckung des Führungsrohres.

Gemäß einer weiteren beispielhaften Ausgestaltung sind die Aussparungen als Langlöcher oder Ellipsen gestaltet, wobei die Aussparungen eine Längserstreckung sowie eine Querstreckung aufweisen, und wobei die Längserstreckung zumindest das Doppelte der Querstreckung beträgt. Dies gilt in einer beispielhaften Ausgestaltung für zumindest einige oder alle der Aussparungen. Die Aussparungen stellen vorzugsweise hinreichend große Öffnungen für das Reinigungsfluid bereit, ohne jedoch die strukturelle Integrität und Stabilität des Führungsrohres deutlich zu verringern.

Gemäß einer weiteren beispielhaften Ausgestaltung weisen die Aussparungen eine Längserstreckung auf, die zwischen 10 mm und 30 mm beträgt, insbesondere zwischen 15 mm und 25 mm, und/oder die Aussparungen eine Querstreckung aufweisen, die zwischen 0,15 mm und 1,0 mm beträgt, insbesondere zwischen 0,25 und 0,4 mm. Auf diese Weise können auch bei miniaturisierten Führungsrohren Aussparungen erzeugt werden, die eine gute Zugänglichkeit für das Reinigungsfluid erlauben.

Gemäß einer weiteren beispielhaften Ausgestaltung weist das Führungsrohr einen Außendurchmesser auf, der kleiner als 2,2 mm ist, vorzugsweise kleiner als 1,8 mm, weiter bevorzugt kleiner als 1,2 mm, und/oder wobei das Führungsrohr eine Wandstärke aufweist, die kleiner 0,3 mm ist, vorzugsweise kleiner als 0,18 mm, weiter bevorzugt kleiner als 0,13 mm. In einer beispielhaften Ausgestaltung beträgt der Außendurchmesser des Führungsrohres 1,0 mm, wobei eine Wandstärke von 0,1 mm gegeben ist. Bei dem Führungsrohr handelt es sich regelmäßig um ein Bauteil auf Basis eines zylindrischen Rohres mit einem konstanten Kreisquerschnitt. Etwaige Aussparungen werden abtragend im Rohr erzeugt.

Der Schaft des Instruments weist regelmäßig auch einen Kreisquerschnitt auf. Jedoch sind auch Ausgestaltungen des Schafts mit einem von einem Kreis abweichenden Querschnitt denkbar.

Gemäß einer weiteren beispielhaften Ausgestaltung sind die Aussparungen nachbearbeitungsarm oder nachbearbeitungsfrei mittels Ultrakurzpulslaserbearbeitung ausgeschnitten. Dies ist vorteilhaft für das Verschleißverhalten und die Leichtgängigkeit beim Betrieb des Instruments, wenn das Steuerelement innerhalb des Führungsrohres entlang der Längserstreckung bewegt wird. Bei den gegebenen miniaturisierten Bauteilen wäre eine Nachbearbeitung der Aussparungen, beispielsweise im Bereich von deren Kanten, mit einem übermäßig hohen Aufwand verbunden.

Die Ultrakurzpulslaserbearbeitung erlaubt eine hochpräzise Herstellung filigraner Strukturen. Ein Vorteil der Ultrakurzpulslaserbearbeitung ist die Eignung für harte Materialien. Ferner erfolgt bei der Ultrakurzpulslaserbearbeitung aufgrund der extrem kurzen Pulse in kürzester Zeit ein hoher Energieeintrag, der dafür sorgt, dass das Material direkt vom festen in den gasförmigen Zustand übergeht und verdampft. Umliegendes Material wird nicht oder nur minimal thermisch beansprucht, wodurch ein hochpräzise Materialabtrag ermöglicht ist. Dieser Vorgang wird als Sublimation bezeichnet.

Die Bearbeitung mittels Ultrakurzpulslaser zur Erzeugung der Aussparungen im Führungsrohr erlaubt auch bei durchmesserkleinen Führungsrohren die Erzeugung einer präzisen Kontur mit hochgenauen Flächen und Kanten. Die Bearbeitung mittels Ultrakurzpulslaser ermöglicht die Erzeugung solcher Aussparungen auch bei Führungsrohren mit einem Durchmesser von weniger als 2,0 mm bis hin zu einem Durchmesser von etwa 1,0 mm oder sogar darunter. Derart durchmesserkleine Rohre können mit konventionellen Laserbearbeitungsverfahren oder anderen abtragenden Bearbeitungsverfahren nicht mit der geforderten Präzision bearbeitet werden.

Gemäß einer weiteren beispielhaften Ausgestaltung sind Kanten der Aussparungen, insbesondere Innenwandkanten, gratarm oder gratfrei gestaltet. Dies ist günstig für die Leichtgängigkeit des Steuerelements beim Betrieb. Ferner kann das Steuerelement im Führungsrohr verschleißarm betrieben werden.

Gemäß einer weiteren beispielhaften Ausgestaltung weisen die Aussparungen Seitenwände auf, wobei einander gegenüberliegende Seitenwände einer Aussparung einen nach außen geöffneten Winkel einschließen, der größer als 5° ist, vorzugsweise größer als 15°, weiter bevorzugt größer als 25°. Der nach außen geöffnete Winkel zeigt mit seiner offenen Seite weg von der Längsachse des Führungsrohres. Gemäß einer weiteren beispielhaften Ausgestaltung sind die Seitenwände radial zu einer Längsachse des Führungsrohres orientiert.

Bei der Bearbeitung mit einem Ultrakurzpulslaser werden die Aussparungen erzeugt, indem Ausschnitte aus der Wandung des Führungsrohres ausgeschnitten werden. Die zueinander geneigte Gestaltung der Seitenwände (beispielsweise einander gegenüberliegende Wände an den Längsseiten eine Aussparung) trägt dazu bei, dass ein vom umliegenden Material des Führungsrohres getrennter Ausschnitt nicht in das Innere des Führungsrohres hineinfällt. Auf diese Weise steigt die Prozesssicherheit bei der Herstellung der Aussparungen, weil die Ausschnitte (Abfallstücke) sicher abgeführt werden können.

Gemäß einer weiteren beispielhaften Ausgestaltung ist die Länge der Innenwandkanten einer Aussparung kleiner als die Länge der Außenwandkanten der Aussparung. Dies gilt jeweils für einen vollständigen Umlauf entlang der Kanten. Auch auf diese Weise kann das Hereinfallen der Ausschnitte verhindert werden. Die Aussparungen sind gemäß einer weiteren beispielhaften Ausgestaltung zumindest abschnittsweise in Richtung auf die Längsachse verjüngt.

Gemäß einer weiteren beispielhaften Ausgestaltung sind am Schaft ein erstes Führungsrohr und ein zweites Führungsrohr angeordnet, die insbesondere außen am Schaft befestigt sind, wobei das erste Führungsrohr ein erstes Steuerelement und das zweite Führungsrohr ein zweites Steuerelement beherbergt, und wobei das erste Steuerelement und das zweite Steuerelement beim distalen Ende mit dem Effektor gekoppelt sind. Auf diese Weise kann der Effektor symmetrisch angesteuert werden. Dies ist etwa dann von Vorteil, wenn entlang des Schafts zwischen den beiden Führungsrohren ein (weiteres) Instrument geführt wird. Insbesondere dann, wenn der Effektor einen Hebel (Albarran-Hebel oder dergleichen) umfasst, ist es günstig, wenn zwei Steuerelemente voneinander beabstandet am Hebel angreifen.

Gemäß einer weiteren beispielhaften Ausgestaltung ist der Schaft mit dem Gehäuse verbunden, wobei der Schaft insbesondere in das Gehäuse mündet, wobei das Gehäuse proximal einen Zugang in den Schaft bereitstellt, und wobei zumindest der Schaft oder das Gehäuse zumindest eine gegenüber einer Schaftachse des Schafts geneigte Bohrung aufweist, durch die ein von der Steuerung des Effektors genutzter Gehäuseraum für Reinigungsmedien zugänglich ist.

Gemäß dieser Gestaltung kann das Reinigungsfluid den Schaft (und das Gehäuse) entlang der Längserstreckung des Schafts durchströmen. Das Gehäuse dient ferner als Träger für Komponenten einer Effektorsteuerung, beispielsweise einen Mechanismus zur Überführung einer Schwenkbewegung (des Betätigungselements) in eine translatorische Bewegung (des Steuerelements). Die gegenüber der Schaftachse geneigten Bohrungen erlauben einen definierten Übertritt des Reinigungsfluids in den Gehäuseraum, damit dort verbaute Komponenten des Instruments gereinigt werden können.

Dies ist etwa dann von Vorteil, wenn das Führungsrohr an seinem proximalen Ende in den Gehäuseraum mündet. Grundsätzlich könnte nämlich darüber der Gehäuseraum kontaminiert werden. Daher ist eine definierte Bohrung im Gehäuse, die den Gehäuseraum zugänglich macht, vorteilhaft, weil das Reinigungsfluid so auf kurzem Wege in das Gehäuse eindringen kann.

Gemäß einer weiteren beispielhaften Ausgestaltung sind das Führungsrohr und der Schaft über den Gehäuseraum fluidisch miteinander verbunden. Mit anderen Worten kann der Gehäuseraum mit dem Reinigungsfluid geflutet werden, wenn der Schaft des Instruments und/oder das Führungsrohr mit dem Reinigungsfluid geflutet werden.

Gemäß einer weiteren beispielhaften Ausgestaltung mündet das Führungsrohr in den Gehäuseraum. Gemäß dieser Ausgestaltung kann sich das Steuerelement in den Gehäuseraum hinein erstrecken, um dort mit Komponenten der Effektorsteuerung gekoppelt zu werden. Über diesen Weg kann grundsätzlich auch eine Verschmutzung des Gehäuseraums erfolgen, die Reinigbarkeit kann über eine Bohrung im Gehäuse verbessert werden.

Gemäß einer weiteren beispielhaften Ausgestaltung ist zumindest eine geneigte Bohrung als Querbohrung durch den Schaft hin zum Gehäuseraum ausgebildet, insbesondere als orthogonal zur Schaftachse des Schafts orientierte Querbohrung. Eine solche Querbohrung durch die Wand des Schafts hin zum Gehäuseraum stellt innerhalb des Gehäuses eine direkte Verbindung in den Gehäuseraum bereit, die für die Reinigung nutzbar ist. Es versteht sich, dass mehrere solcher Querbohrungen vorgesehen sein können.

Gemäß einer weiteren beispielhaften Ausgestaltung ist zumindest eine geneigte Bohrung als Verbindungsbohrung zwischen einem Gehäuseteil beim proximalen Ende des Schafts und dem Gehäuseraum ausgebildet, insbesondere unter einem spitzen Winkel zur Längsachse des Schafts, der nach distal geöffnet ist.

Im Gehäuse steht üblicherweise ein Sitz für das proximale Ende des Schafts bereit. Die geneigte Bohrung kann sich durch diesen Sitz hindurch in Richtung auf den Gehäuseraum erstrecken, ohne den Schaft selbst zu durchdringen. Mehrere solcher Bohrungen können um den Schaft verteilt sein und den Gehäuseraum zugänglich machen. Beispielhaft handelt es sich um geneigte Bohrung, die sich ausgehend von einer proximalen Öffnung des Gehäuses hin zum Gehäuseraum erstreckt. Die Mündung der geneigten Bohrung im Gehäuse ist beispielsweise in einem Abschnitt, der einerseits proximal gegenüber dem proximalen Ende des Schafts und andererseits distal gegenüber einem distalen Ende eines Reinigungsadapters versetzt, der in eine proximale Aufnahme im Gehäuse eingesetzt ist.

Gemäß einer weiteren beispielhaften Ausgestaltung ist das Steuerelement an seinem proximalen Ende mit einer Stange gekoppelt, die sich durch ein Wandstück in das Gehäuse erstreckt, wobei ein proximales Ende des Führungsrohres vom Gehäuse beabstandet ist. Diese Gestaltung hat den Vorteil, dass weder das Führungsrohr noch das Steuerelement in den Gehäuseraum münden bzw. sich in den Gehäuseraum hinein erstrecken.

Gemäß einem weiteren Aspekt bezieht sich die vorliegende Offenbarung auf ein Verfahren zur Herstellung eines Führungsrohres eines medizinischen Instruments, insbesondere eines Instruments gemäß zumindest einer der hierin beschriebenen Ausgestaltungen, mit den folgenden Schritten:
- Bereitstellung eines Werkstücks in Form eines Halbzeugs mit einem dünnwandigen Rohrkörper mit einer Längsachse, der sich zwischen einem distalen Ende und einem proximalen Ende erstreckt,
- Bereitstellung eines Ultrakurzpulslasers,
- Einspannen des Werkstücks in einem Werkstückhalter,
- Betreiben des Ultrakurzpulslasers zur Bearbeitung des Werkstücks mittels Sublimation,
- Erzeugung einer Mehrzahl von Aussparungen im Werkstück, umfassend:
   - Erzeugen einer koordinierten Relativbewegung zwischen dem Rohrkörper und dem Ultrakurzpulslaser bei aktiviertem Ultrakurzpulslaser, umfassend eine translatorische Bewegung entlang der Längsachse und eine Schwenkbewegung mit Bezug auf die Längsachse zur Erzeugung eines Ausschnitts im Werkstück, der eine Aussparung definiert, und
   - Erzeugen einer Vorschubbewegung zwischen dem Rohrkörper und dem Ultrakurzpulslaser bei deaktiviertem Ultrakurzpulslaser, umfassend eine translatorische Bewegung entlang der Längsachse, um mehrere Aussparungen zu erzeugen, die voneinander beabstandet sind.

Auch auf diese Weise wird die Aufgabe der Offenbarung gelöst.

Durch Bearbeitung mit einem Ultrakurzpulslaser können auch in miniaturisierten Werkstücken besonders filigrane Aussparungen erzeugt werden. Dies kann nachbearbeitungsarm oder nachbearbeitungsfrei erfolgen. Eine etwaige Nachbearbeitung wäre angesichts der geringen Abmessungen der Werkstücke (Rohre mit Durchmesser von beispielsweise 2,0 mm oder 1,0 mm) mit hohem Aufwand verbunden, so dass die Bearbeitung mit dem Ultrakurzpulslaser Zusatzaufwand vermeidet. Durch den Ultrakurzpulslaser wird Material sublimiert, so dass im Rahmen der Relativbewegung zwischen dem Ultrakurzpulslaser und dem Werkstück Ausschnitte im Werkstück erzeugt werden, die die Aussparungen freigeben.

Zu Erzeugung der Aussparung wird also nicht umfänglich Material abgetragen. Stattdessen wird eine relativ schmale Schnittfuge zwischen den Ausschnitten und dem umliegenden Material der Rohre erzeugt.

Die Bearbeitung mit dem Ultrakurzpulslaser hat als weiteren Vorteil nur einen relativ geringen Wärmeeintrag in das Werkstück. Daher wird das Gefüge im Werkstück nicht übermäßig beeinträchtigt.

Die Relativbewegung zwischen dem Werkstück und dem Ultrakurzpulslaser kann bei einem stillstehenden Laser durch eine Bewegung des Werkstücks in Bezug auf den Laser erzeugt werden. Die Relativbewegung zwischen dem Werkstück und dem Ultrakurzpulslaser kann bei einem stillstehenden Werkstück durch eine Bewegung des Lasers in Bezug auf das Werkstück erzeugt werden. Auch eine kombinierte Bewegung von Werkstück und Laser ist vorstellbar.

Gemäß einer beispielhaften Ausgestaltung weist das Verfahren ferner die folgenden Schritte auf:
- Abführen der Ausschnitte radial nach außen, wobei die Aussparungen zueinander geneigte und zueinander gegenüberliegende Seitenwände aufweisen, deren Neigungswinkel in Richtung der Längsachse zeigt, und/oder
- Rotieren des Werkstücks um die Längsachse, bis die Ausschnitte schwerkraftunterstützt abführbar sind.

Es ist vorteilhaft, wenn einander gegenüberliegende Seitenwände der Aussparungen zueinander geneigt sind, so dass die erzeugten Ausschnitte nicht nach innen in das Werkstück hineinfallen können. Die Aussparungen sind allgemein zumindest abschnittsweise in Richtung auf die Längsachse verjüngt. Dies kann eine radiale Orientierung der Seitenwände umfassen. Dies erleichtert das Abführen der Ausschnitte. Gegebenenfalls genügt eine Rotation des Werkstücks (in horizontaler Orientierung) um dessen Längsachse, bis die Ausschnitte nach unten herausfallen können.

Ein offenbarungsgemäß gestaltetes und/oder hergestelltes Führungsrohr kann zur Führung eines Steuerelements für ein medizinisches Instrument genutzt werden, beispielsweise für einen Arbeitseinsatz mit einem auslenkbaren Effektor beim distalen Ende. Das Führungsrohr kann mit einem Schaft des Instruments verbunden werden, um eine Führung für ein mit dem Effektor gekoppeltes Steuerelement bereitzustellen. Das Führungsrohr kann innerhalb des Schafts angeordnet sein. Das Führungsrohr kann außerhalb des Schafts angeordnet sein. Üblicherweise erstreckt sich das Führungsrohr parallel zum (starren) Schaft.

Bei der Reinigung eines solchen Instruments gestatten die Aussparungen einen Eintritt/Austritt des Reinigungsfluids zur Reinigung des Steuerelements und damit gekoppelter Komponenten.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Offenbarung zu verlassen.

Weitere Merkmale und Vorteile der Erfindung ergeben sich aus der nachfolgenden Beschreibung und Erläuterung mehrerer beispielhafter Ausführungsformen unter Bezugnahme auf die Zeichnungen. Es zeigen:
- Fig. 1:: eine schematische Ansicht eines medizinischen Systems in Form eines mehrteiligen Instruments, mit voneinander gelösten Komponenten;
- Fig. 2:: eine perspektivische Teilansicht eines Instruments mit einem auslenkbaren Hebel;
- Fig. 3:: eine perspektivische Teilansicht eines Führungsrohres des Instruments gemäß Fig. 2;
- Fig. 4:: eine Schnittansicht zur Veranschaulichung eines Querschnitts des Führungsrohres gemäß Fig. 3;
- Fig. 5:: eine seitliche Teilansicht des Führungsrohres gemäß Fig. 4;
- Fig. 6:: eine perspektivische, geschnittene Teilansicht eines Instruments bei einem proximalen Ende eines Schafts, der in ein Gehäuse mündet;
- Fig. 7: eine perspektivische, geschnittene Teilansicht eines weiteren Instruments bei einem proximalen Ende eines Schafts, der in ein Gehäuse mündet;
- Fig. 8: eine schematische Ansicht einer Fertigungsvorrichtung zur Bearbeitung eines Werkstücks zur Herstellung eines mit Aussparungen versehenen Führungsrohres;
- Fig. 9: eine perspektivische schematische Ansicht des Werkstücks gemäß Fig. 8 zur Veranschaulichung einer Relativbewegung bei der Bearbeitung;
- Fig. 10: eine schematische Seitenansicht eines durch die Bearbeitung gewonnenen Führungsrohres mit Ausschnitten, die Aussparungen freigeben; und
- Fig. 11:: ein vereinfachtes Blockdiagramm zur Veranschaulichung einer beispielhaften Ausgestaltung eines Verfahrens zur Herstellung eines Führungsrohres eines medizinischen Instruments.

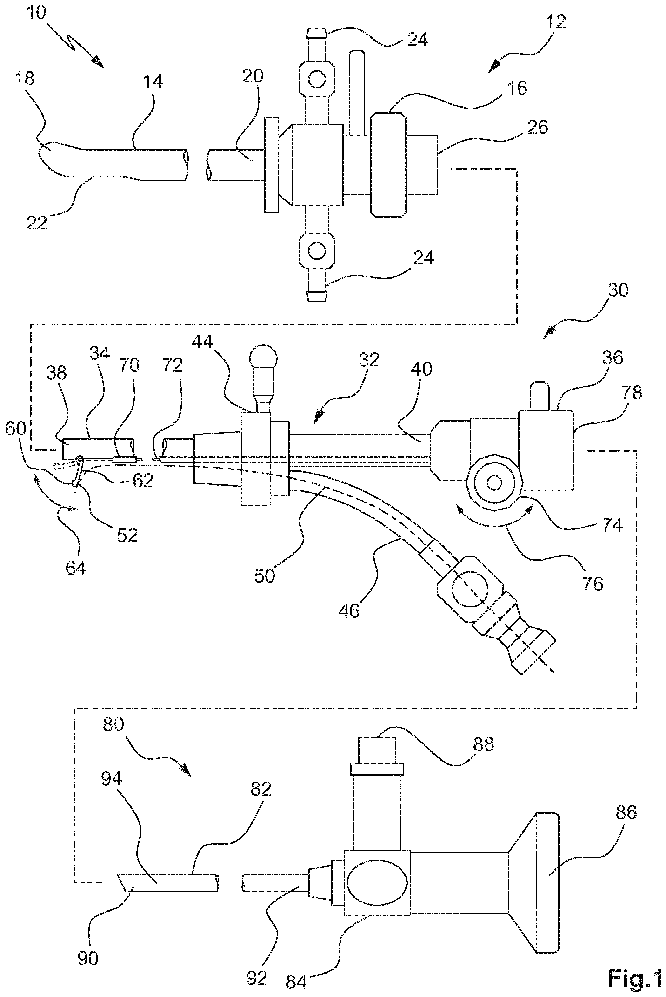

Fig. 1 veranschaulicht anhand einer schematischen Ansicht einer insgesamt mit 10 bezeichnetes medizinisches System in einem Zustand, in dem dessen Komponenten nicht miteinander gefügt sind. Das medizinische System 10 kann auch als mehrteiliges Instrument bezeichnet werden, da es aus verschiedenen Komponenten zusammengesetzt ist, die wiederum als Instrument bezeichnet werden können. Das System 10 ist beispielsweise als Zystoskop gestaltet.

Das System 10 umfasst ein Schaftteil 12, das beispielsweise als Zystoskopschaft gestaltet ist. Das Schaftteil 12 umfasst einen Außenschaft 14 und ein Gehäuse 16. Der Außenschaft erstreckt sich zwischen einem distalen Ende 18 und einem proximalen Ende 20. Beim distalen Ende 18 ist eine Öffnung 22 in den Außenschaft 14 eingebracht. Am Gehäuse 16 des Schaftteils 12 sind im Ausführungsbeispiel Spülanschlüsse 24 zur Durchleitung und Steuerung eines Spülfluids ausgebildet. Das Gehäuse 16 weist proximal eine Aufnahme 26 auf, in die ein weiteres Instrument 30 in das Schaftteil 12 eingesteckt werden kann.

Bei dem Instrument 30 handelt es sich beispielhaft um einen Arbeitseinsatz 32. Der Arbeitseinsatz 32 umfasst einen Schaft 34 und ein Gehäuse 36. Der Schaft 34 erstreckt sich zwischen einem distalen Ende 38 und einem proximalen Ende 40. Das Gehäuse 36 sitzt beim proximalen Ende 40 des Schafts 34. Entlang der Längserstreckung des Schafts 34 zwischen dem proximalen Ende 40 und dem distalen Ende 38 sitzt eine Brücke 44. Das Instrument 30 kann mit dem Schaft 34 in die Aufnahme 26 beim Gehäuse 16 des Schaftteils 12 eingeführt werden. Die Brücke 44 kann mit der Aufnahme 26 gekoppelt werden.

Das Instrument 30 weist ferner einen mit der Brücke 44 gekoppelten Arbeitskanal 46 auf. Über den Arbeitskanal 46 kann ein flexibles Instrument 50 (in Fig. 1 durch eine strichpunktierte Linie angedeutet) eingeführt werden. Ein solches flexibles Instrument 50 kann dann gemeinsam mit dem Schaft 34 in dem Außenschaft 14 angeordnet sein. Das flexible Instrument 50 kann bei der Öffnung 22 aus dem Außenschaft 14 hervortreten.

Das flexible Instrument 50 weist ein distales Ende 52 auf, das durch einen hier als Hebel 62 gestalteten Effektor 60 auslenkbar ist. Vergleiche hierzu auch den gekrümmten Doppelpfeil 64. Der Hebel 62 ist beispielhaft als sogenannter Albarran-Hebel gestaltet. Der Hebel 62 ist zwischen zwei Positionen relativ zum Schaft 34 verlagerbar, vergleiche die gestrichelte Darstellung in Fig. 1. Auf diese Weise kann das distale Ende 52 des flexiblen Instruments 50 in einer ersten Stellung an den Schaft 34 angeschmiegt und in einer zweiten Stellung gegenüber dem Schaft 34 ausgelenkt sein.

Der Effektor 60 kann auch anderweitig gestaltet sein, es muss sich nicht um einen Hebel 62 handeln. Der Effektor 60 ist über ein in einem Führungsrohr 70 geführtes Steuerelement 72 steuerbar. Im Ausführungsbeispiel erstreckt sich das Führungsrohr 70 vom distalen Ende 38 (distaler Endbereich) hin zum proximalen Ende 40 des Schafts 34. Gleichermaßen erstreckt sich das Steuerelement 72 zwischen dem distalen Ende 38 und dem proximalen Ende 40 des Schafts 34. Beim proximalen Ende 40 des Schafts 34 ist das Steuerelement 72 mit einem Betätigungselement 74 gekoppelt. Im Ausführungsbeispiel gemäß Fig. 1 ist das Betätigungselement 74 als Rad gestaltet, auch eine Gestaltung als Hebel ist denkbar. Durch eine Bewegung des Betätigungselement 74 (vergleiche den gekrümmten Doppelpfeil 76) kann das Steuerelement 72 bewegt werden, um den Effektor 60 zu betätigen, vergleiche den gekrümmten Doppelpfeil 64).

Beim proximalen Ende des Gehäuses 36 des Instruments 30 ist eine Aufnahme 78 ausgebildet, in die ein weiteres Instrument einführbar ist, im Ausführungsbeispiel ein Endoskop 80.

Das Endoskop 80 weist einen Endoskopschaft 82 und ein Gehäuse 84 auf. Das Gehäuse 84 trägt proximal ein Okular 86, das im Betrieb eine für das menschliche Auge wahrnehmbare optische Abbildung bereitstellt. Es versteht sich, dass in alternativen Ausgestaltungen auch Endoskope mit Kameraaufsatz statt Okular 86 verwendbar sind. Schließlich sind auch Endoskope bekannt, bei denen die Bilderfassung durch einen Bildsensor erfolgt, beispielsweise durch einen distalen Bildsensor, ohne dass eine optische Abbildung im Gehäuse 84 bereitgestellt wird. Das Endoskop 80 gemäß Fig. 1 weist beim Gehäuse 84 ferner einen Lichtanschluss 88 auf. Beispielhaft kann ein Lichtleitkabel an den Lichtanschluss 88 angekoppelt werden, um das Sichtfeld auszuleuchten. Andere Beleuchtungstechnik sind grundsätzlich vorstellbar, beispielsweise die Beleuchtung durch integrierte Lichtquellen.

Der Endoskopschaft 82 erstreckt sich zwischen einem distalen Ende 90 und einem proximalen Ende 92 und beherbergt eine Beobachtungsoptik 94. Das Endoskop 80 ist beispielhaft als Instrument mit geneigter Blickrichtung gestaltet. Es sind jedoch auch Endoskope mit Geradeausblick bekannt. Ein konventionell gestaltetes Endoskop 80 weist im Endoskopschaft 82 einen Beobachtungsstrahlengang und einem Beleuchtungsstrahlengang auf.

Die in Fig. 1 gezeigten Instrumente (Schaftteil 12, Arbeitseinsatz 32, Endoskop 80 und gegebenenfalls flexibles Instrument 50) können miteinander kombiniert werden, um ein mehrteiliges Instrument (medizinisches System 10) zu bilden. Auf diese Weise kann beispielsweise ein Zystoskop, Hysteroskop und/oder Resektoskop bereitgestellt werden. Die Möglichkeit zur Auslenkung mit dem Effektor 60 vergrößert den Anwendungsbereich für ein solches mehrteiliges Instrument. Dies erfordert jedoch die Integration zumindest eines Steuerelements 72, das sich durch ein Führungsrohr 70 von distal nach proximal erstreckt. Das Steuerelement 72 und das Führungsrohr 70 sind in einem Bereich verbaut, in dem es auf den beanspruchten Bauraum ankommt. Um die Belastung für den Patienten insgesamt zu minimieren sollte das System 10 im Bereich des Außenschafts 14 einen möglichst kleinen Außendurchmesser aufweisen. Dieses Ziel lässt sich nur dann erreichen, wenn auch die in den Außenschaft 14 einzuführenden Komponenten nur sehr kleine Querschnitte aufweisen. Dies führt jedoch im Umkehrschluss zu Herausforderungen bei der Reinigung solcher Komponenten.

Beispielhaft ist die Reinigung des als Arbeitseinsatz 32 gestalteten Instruments 30 aufwendig, weil auch die Mechanik zur Steuerung des Effektors 60 kontaminiert sein kann. Daher sollte auch das Führungsrohr 70 mit dem darin geführten Steuerelement 72 prozesssicher gereinigt werden können.

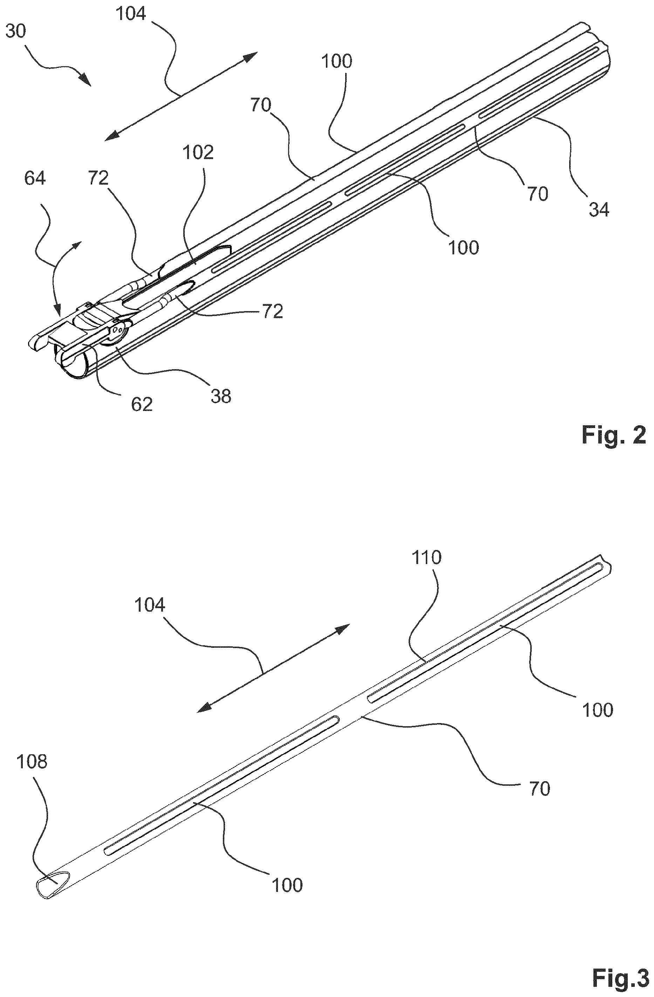

Mit Bezugnahme auf die Figuren 2 und 3 wird anhand perspektivischer Teildarstellungen eine beispielhafte Ausgestaltung des Instruments 30 veranschaulicht. Ergänzend wird auf die Gestaltung gemäß Fig. 1 verwiesen, wobei für entsprechende Teile entsprechende Bezugsziffern verwendet werden. Das Instrument 30 gemäß Fig. 2 kann grundsätzlich als Arbeitseinsatz 32 (vergleiche Fig. 1) verwendet werden. In Fig. 2 ist das distale Ende 38 des Schafts 34 und ein sich anschließender Bereich dargestellt. Beim distalen Ende 38 ist der auslenkbarer Hebel 62 (Albarran-Hebel) gelagert, vergleiche zur Veranschaulichung der Auslenkbewegung den gekrümmten Doppelpfeil 64.

Zur Steuerung des Hebels 62 dienen zwei Steuerelemente 72, die jeweils in einem Führungsrohr 70 geführt sind. Im Ausführungsbeispiel gemäß Fig. 2 sind demgemäß zwei Führungsrohre 70 an der Außenseite des Schafts 34 befestigt. Die Führungsrohre 70 erstrecken sich parallel zum Schaft 34. Mit anderen Worten weisen im Ausführungsbeispiel der Schaft 34 und die Führungsrohr 70 eine zueinander parallele Längserstreckung (vergleiche den Doppelpfeil 104) auf. Die Führungsrohre 70 sind jeweils entlang ihrer Längserstreckung 104 mit Aussparungen 100 versehen, durch die das Innere der Führungsrohres 70 zugänglich ist. Der Schaft 34 ist ferner an seiner distalen Stirn geöffnet. Ferner ist am Umfang des Schafts 34 beim auslenkbaren Hebel 62 eine Öffnung 102 im Schaft 34 ausgebildet.

Fig. 3 zeigt eine auf der Darstellung gemäß Fig. 2 beruhende, vergrößerte Ansicht eines Führungsrohres 70. Das Führungsrohr 70 ist entlang seiner Längserstreckung 104 mit mehreren Aussparungen 100 versehen, die eine Reihe 110 bilden. Das Führungsrohr 70 weist ferner eine distale Öffnung 108 auf, durch die das Steuerelement 72 (vergleiche Fig. 2) aus dem Führungsrohr 70 hervortritt. Die Aussparungen 100 sind im Ausführungsbeispiel als Langlöcher gestaltet. Durch die Aussparungen 100 kann Reinigungsfluid in die Führungsrohr 70 einströmen oder daraus herausströmen. Mit anderen Worten kann also auch das Steuerelement 72 in dem Bereich seiner Längserstreckung (Pfeil 104) gut gereinigt werden, in dem es innerhalb des Führungsrohres 70 angeordnet ist.

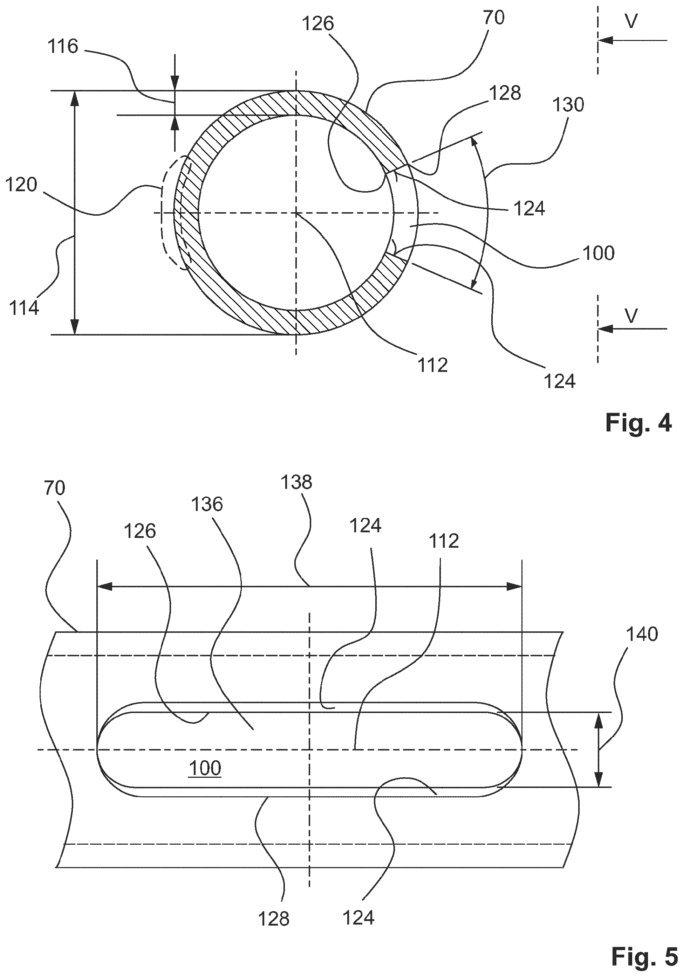

Mit ergänzender Bezugnahme auf die Figuren 4 und 5 werden denkbare Gestaltungen und Abmessungen des Führungsrohres 70 und der Aussparungen 100 veranschaulicht. Fig. 4 zeigt einen Querschnitt durch das Führungsrohr 70 im Bereich einer Aussparung 100. Fig. 5 veranschaulicht anhand einer Teilansicht eine Seitenansicht des Führungsrohres 70 mit einer Frontalansicht auf eine als Langloch gestaltete Aussparung 100. Die Ansichtsorientierung von Fig. 5 ergibt sich aus der Linie V-V in Fig. 4. Eine Längsachse 112 durch das Führungsrohr 70 ist in Fig. 5 parallel zur Ansichtsebene. In Fig. 4 ist die Längsachse 112 senkrecht zur Ansichtsebene.

Das Führungsrohr 70 weist einen Außendurchmesser 114 und eine Wandstärke 116 auf. Der Außendurchmesser 114 beträgt beispielsweise 1,0 mm. Die Wandstärke 116 beträgt beispielsweise 0,1 mm. Andere Werte sind grundsätzlich denkbar, das Führungsrohr 70 sollte jedoch möglichst klein sein, um so wenig Platz wie möglich im medizinischen System 10 zu beanspruchen. Bei einem derart miniaturisierten Führungsrohr 70 können die Aussparungen 100 nicht oder nur mit sehr hohem Aufwand mit etablierten Fertigungstechniken erzeugt werden. Eine Fertigungstechnologie, die sich zur Herstellung der Aussparungen 100 in filigran gestalteten Führungsrohren 72 eignet, ist das sogenannte Ultrakurzpulslasern.

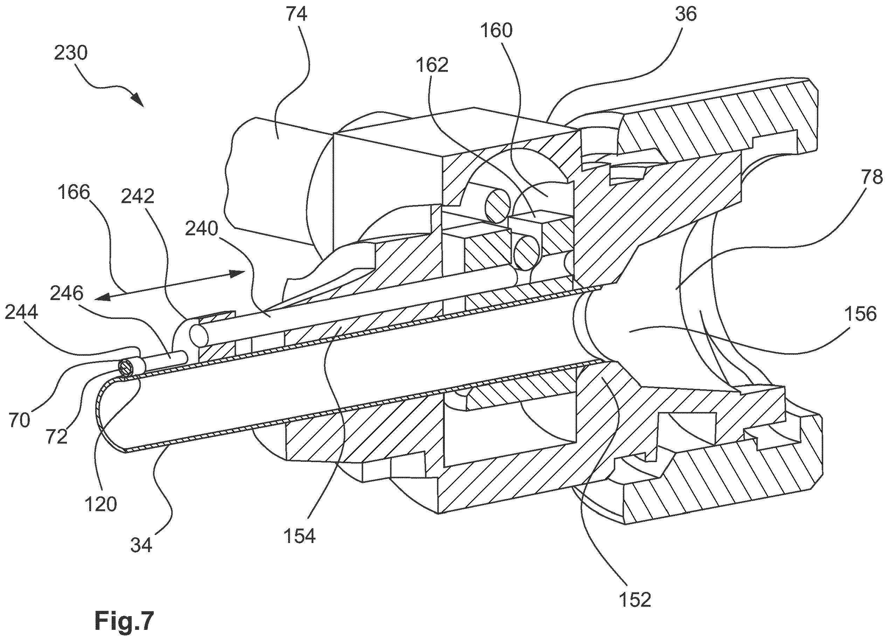

Die Aussparung 100 ist in einem Umfangsabschnitt am Führungsrohr 70 angeordnet, der einem Umfangsabschnitt 120 gegenüberliegt, in dem das Führungsrohr 70 mit dem Schaft 34 verbunden ist, vergleiche hierzu auch Fig. 2 und Fig. 7.

Die in Fig. 4 im Schnitt gezeigte Aussparung 100 weist Seitenwände 124 auf. Ferner sind Kanten (Innenkanten) 126 und Kanten (Außenkanten) 128 vorgesehen. In einer beispielhaften Ausgestaltung sind die Seitenwände 124 radial zur Längsachse 112 orientiert. Mit anderen Worten sind die beiden in der Darstellung gemäß Fig. 4 gezeigten und einander gegenüberliegenden Seitenwände 124 nicht parallel zueinander sondern zueinander V-förmig geneigt. Ein Öffnungswinkel ist in Fig. 4 mit 130 angedeutet. Mittels Ultrakurzpulslasern können die Aussparungen 100 hochpräzise erzeugt werden. Beispielhaft können die Kanten 126, 128 gratfrei oder nahezu gratfrei erzeugt werden. Insgesamt können die Aussparungen 100 möglichst nachbearbeitungsfrei oder nachbearbeitungsarm mittels Ultrakurzpulslasern erzeugt werden.

Fig. 5 veranschaulicht die Gestaltung der in Fig. 4 im Schnitt gezeigten Aussparung 100 weiter. Aus der Neigung der gegenüberliegenden Seitenwände 124 in Fig. 4 ergibt sich eine Gestaltung, bei der eine Gesamtlänge der Innenkanten 126 kürzer als die Gesamtlänge der Außenkanten 128 ist. Mit anderen Worten ist die Aussparung 100 an ihrer Innenseite schmaler als an ihrer Außenseite. Dies hat den Vorteil, dass etwaige Ausschnitte, die bei der Erzeugung der Aussparung 100 vom umliegenden Material des Führungsrohres 70 getrennt werden, nicht in das Innere des Führungsrohres 70 hineinfallen können. Einander gegenüberliegende Seitenwände 124 der Aussparung 100 müssen nicht notwendigerweise entlang eines gesamten Umlaufs ununterbrochen zueinander V-förmig geneigt sein. Die Aussparung 100 ist in Richtung auf die Längsachse 112 verjüngt.

Die Aussparung 100 ist in Fig. 5 als Langloch 136 gestaltet. Demgemäß weist die Aussparung 100 eine Längserstreckung 138 auf, die ein Mehrfaches der Quererstreckung 140 beträgt. Die Längserstreckung 138 beträgt beispielsweise zwischen 10 mm und 30 mm. Die Quererstreckung 140 beträgt beispielsweise zwischen 0,25 mm und 0,4 mm. Die Darstellung in Fig. 5 ist daher nicht notwendigerweise maßstabsgetreu.

Die in Fig. 4 und Fig. 5 gezeigte Gestaltung der Aussparung 100 lässt sich beispielsweise erzeugen, wenn der Ultrakurzpulslaser senkrecht zur Längsachse 112 orientiert und radial auf die Längsachse 112 ausgerichtet ist, wobei eine Relativbewegung (kombinierte Linearbewegung und Rotationsbewegung) zwischen dem Führungsrohr 70 und dem Ultrakurzpulslaser erzeugt wird, unter Beibehaltung der radialen Ausrichtung zur Längsachse 112. Auf diese Weise wird das Abführen etwaiger Ausschnitte bei der Herstellung der Aussparungen 100 erleichtert. Es ergeht sich, dass auch andere Konturen als die Langloch-Gestaltung gemäß Fig. 5 denkbar sind, etwa eine ovale Gestaltung der Aussparungen 100.

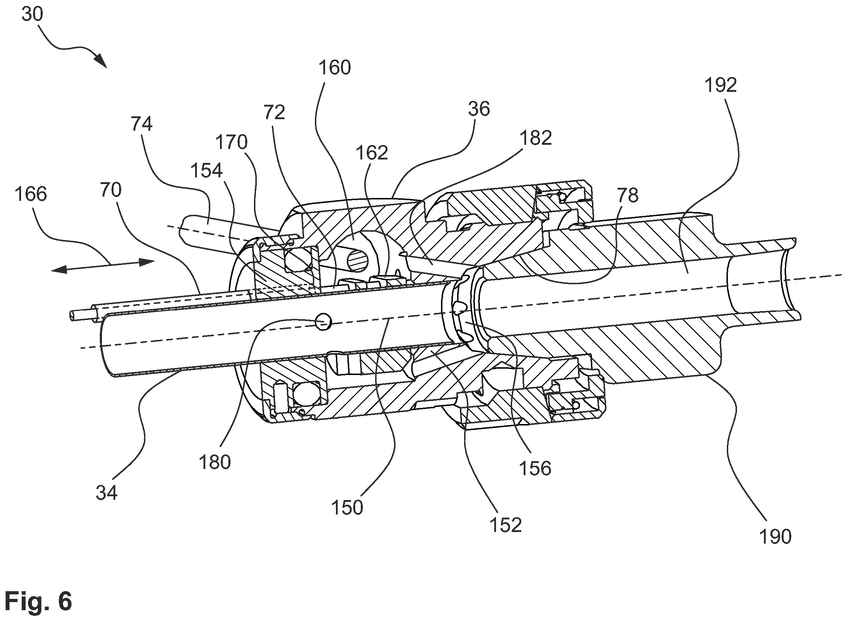

Mit Bezugnahme auf Fig. 6 wird anhand einer perspektivischen Schnittansicht eine beispielhafte Ausgestaltung des Instruments 30 im Bereich von dessen Gehäuse 36 veranschaulicht. In grundsätzlich zuvor schon beschriebener Weise kann das Instrument 30 mit Führungsrohren 70 versehen sein, die seitliche Aussparungen 100 aufweisen (vergleiche Fig. 3).

Der Schaft 34 des Instruments 30 mündet in das Gehäuse 36. Durch den Schaft 34 erstreckt sich eine Schaftachse 150, die eine Haupterstreckungsrichtung für den Schaft 34 beschreibt. Der Schaft 34 ist ein starrer Schaft und besteht aus einem geeigneten Metall, beispielsweise aus chirurgischem Edelstahl. Im Gehäuse 36 ist ein Gehäuseteil 152 ausgebildet, das als Sitz für den Schaft 34 dient. Der Schaft 34 erstreckt sich durch ein dem Gehäuse 36 zugeordnetes Wandstück 154 in das Gehäuse 36 hinein. Das Gehäuse 36 stellt am proximalen Ende des Schafts 34 einen Zugang 156 in den Schaft 34 bereit.

Im Gehäuse 36 ist ein den Schaft 34 zumindest teilweise umgebender Gehäuseraum 160 ausgebildet, der eine Effektorsteuerung 162 beherbergt. Die Effektorsteuerung 162 koppelt das Betätigungselement 74 (vergleiche auch Fig. 1) mit dem Steuerelement 72 zur Steuerung des Effektors 60 (vergleiche wiederum Fig. 1). Auf diese Weise kann eine Betätigung des Betätigungselements 74 in eine Bewegung des Steuerelements 72 nach proximal oder nach distal überführt werden, vergleiche den Doppelpfeil 166.

Im Ausführungsbeispiel gemäß Fig. 6 erstreckt sich das Steuerelement 72 in den Gehäuseraum 160 hinein. Das Führungsrohr 70, durch das sich das Steuerelement 72 erstreckt, erstreckt sich durch das Wandstück 154 und mündet mit seinem proximalen Ende 170 in den Gehäuseraum 160. Über diesen Pfad (Bewegung 166 des Steuerelements 72 im Führungsrohr 70) kann der Gehäuseraum 160 verschmutzt werden. Das Eindringen eines Reinigungsfluids wird jedoch durch einen vergleichsweise kleinen Spalt zwischen dem Steuerelement 72 und dem Führungsrohr 70 erschwert.

Daher ist es vorstellbar, Bohrungen 180, 182 in den Gehäuseraum 160 bereitzustellen, über die Reinigungsfluid leicht in den Gehäuseraum 162 eindringen kann. Im Ausführungsbeispiel gemäß Fig. 6 sitzt ein Reinigungsadapter 190 in der Aufnahme 78 beim proximalen Ende des Gehäuse 36, vergleiche auch Fig. 1. Über den Reinigungsadapter 190 wird ein Durchlass 192 bereitgestellt, über den ein Reinigungsfluid über den Zugang 156 in den Schaft 34 eindringen kann. Im Bereich des Gehäuseraums 160 weist der Schaft 34 eine oder mehrere geneigte Bohrungen in Form von Querbohrungen 180 auf, die im Ausführungsbeispiel orthogonal zur Schaftachse 150 orientiert sind. Alternativ oder zusätzlich sind geneigte Bohrungen 182 durch das als Sitz für den Schaft 34 dienende Gehäuseteil 152 vorgesehen, die ebenso in den Gehäuseraum 160 münden. Die (Mündungen der) geneigten Bohrungen 182 sind im Bereich der Aufnahme 78 dem Schaft 34 proximal vorgelagert. Die geneigten Bohrungen 182 sind im Ausführungsbeispiel spitzwinklig gegenüber der Schaftachse 150 orientiert, wobei der sich ergebende Neigungswinkel nach distal geöffnet ist.

Die Bohrungen 180, 182 können alternativ zueinander oder in Kombination eingebracht sein, um das Fluten des Gehäuseraums 160 mit dem Reinigungsfluid zu vereinfachen.

Mit Bezugnahme auf Fig. 7 wird eine gegenüber der Darstellung in Fig. 6 abgewandelte Ausgestaltung eines mit 230 bezeichneten Instruments veranschaulicht. Das Instrument 230 ist grundsätzlich ähnlich dem Instrument 30 gestaltet und als Arbeitseinsatz verwendbar. Daher wird nachfolgend zuvorderst auf abweichend gestaltete Merkmale eingegangen. Im Übrigen werden für entsprechende Teile entsprechende Bezugsziffern verwendet. Der Schaft 34 mündet in das Gehäuse 36, wobei das proximale Ende des Schafts 34 in einem als Sitz dienenden Gehäuseteil 152 ruht.

Das Gehäuse 36 stellt proximal eine Aufnahme 78 bereit (vergleiche Fig. 1 und Fig. 6), über die ein Zugang 156 in den Schaft 34 zugänglich ist. Im Gehäuse 36 ist ein Gehäuseraum 160 ausgebildet, der den Schaft 34 zumindest teilweise umgibt und eine Effektorsteuerung 162 beherbergt. Das Ausführungsbeispiel des Instruments 230 gemäß Fig. 7 unterscheidet sich von der Gestaltung des Instruments 30 gemäß Fig. 6 zumindest dahingehend, dass sich das Steuerelement 72 nicht bis in den Gehäuseraum 160 erstreckt. Das Führungsrohr 70 kann analog zu den zuvor beschriebenen Ausgestaltungen mit Aussparungen 100 versehen sein.

Das Führungsrohr 70 weist ein proximales Ende 244 auf, das vom Gehäuse 36 beabstandet ist. Auch das Steuerelement 72 ragt nicht in das Gehäuse 36 beziehungsweise in den Gehäuseraum 160 hinein. Stattdessen ist eine Stange 240 vorgesehen, die die Effektorsteuerung 162 innerhalb des Gehäuseraums 160 mit dem Steuerelement 72 und schlussendlich mit dem Effektor 60 (vergleiche Fig. 1) koppelt. Im Ausführungsbeispiel erstreckt sich die Stange 240 durch das Wandstück 154 in den Gehäuseraum 160 hinein. Das Steuerelement 72 ragt mit seinem proximalen Ende 246 zumindest zeitweise nach proximal aus dem Führungsrohr 70 heraus. Das proximale Ende 246 des Steuerelements 72 ist mit einem Verbinder 242 gekoppelt, der auch als Verbindungsnippel bezeichnet werden kann. Über den Verbinder 242 wird die Bewegung 166 nach proximal/distal auf das Steuerelement 72 übertragen. Wenn die Stange 240 hinreichend dicht im Wandstück 154 geführt ist, droht keine störende Kontamination des Gehäuseraums 160. Das im Führungsrohr 70 gelagerte Steuerelement 72 kann auch im Bereich von seinem proximalen Ende 246 außerhalb des Gehäuse 36 gereinigt werden.

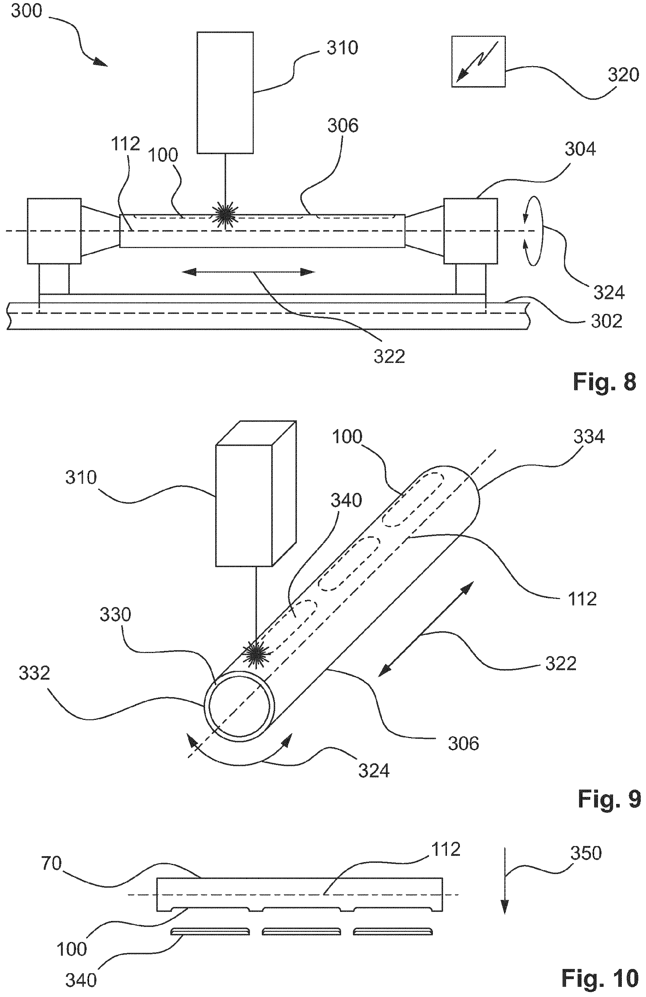

Mit Bezugnahme auf die Figuren 8-10 wird ein denkbarer Ansatz zur Fertigung von mit Aussparungen 100 versehenen Führungsrohren 70 veranschaulicht.

Fig. 8 veranschaulicht anhand einer schematischen Darstellung eine insgesamt mit 300 bezeichnete Fertigungsvorrichtung. Die Fertigungsvorrichtung 300 umfasst im Ausführungsbeispiel eine Basis 302, die einen Werkstückhalter 304 zur Aufnahme eines rohrförmigen Werkstücks 306 trägt. Die Fertigungsvorrichtung 300 umfasst einen Ultrakurzpulslaser 310, der auf das Werkstück 306 einwirken kann, um dort Material abzutragen bzw. Schnitte zu erzeugen.

Ferner ist eine Steuereinrichtung 320 Bestandteil der Fertigungsvorrichtung 300, mit der Steuereinrichtung 320 kann eine Relativbewegung zwischen dem Ultrakurzpulslaser 310 und dem Werkstück 306 koordiniert werden, um die Aussparungen 100 zu erzeugen. Die koordinierte Relativbewegung umfasst beispielhaft eine translatorische Bewegung 322 entlang der Längsachse 112 (beispielsweise relativ zur Basis 302) sowie eine Schwenkbewegung 324 um die Längsachse 312 des Werkstücks 306.

Fig. 9 veranschaulicht ergänzend anhand einer perspektivischen schematischen Darstellung das Zusammenwirken zwischen dem Ultrakurzpulslaser 310 und dem hier als Rohrkörper 330 gestalteten Werkstück 306. Mit dem in Fig. 8 gezeigten Werkstückhalter 304 kann das Werkstück 306 zwischen einem proximalen Ende 334 und einem distalen Ende 332 eingespannt und um seine Längsachse 112 rotiert werden.

Das Werkstück 306 kann translatorisch (Pfeil 322) und rotatorisch (Schwenkbewegung 324) bewegt werden, um bei feststehendem Ultrakurzpulslaser 310 Ausschnitte 340 im Werkstück 306 zu erzeugen, die die Aussparung 100 (vergleiche Fig. 10) definieren und freigeben. Die Aussparungen 100 sind beispielhaft analog zur Gestaltung gemäß den Figuren 4 und 5 gestaltet. Diese Gestaltung hat den Vorteil, dass die Ausschnitte 340, die die Aussparungen 100 freigeben, nicht in das Innere des Werkstücks 106 bzw. des daraus erzeugten Führungsrohres 70 hineinfallen können. Fig. 10 veranschaulicht anhand einer schematisch stark vereinfachten Darstellung, dass das Führungsrohr 70 um seine Längsachse 112 rotiert werden kann, so dass die Ausschnitte 340 aus den Aussparungen 100 herausfallen können. Mit anderen Worten kann das Abführen der Ausschnitte 340 unterstützt durch Schwerkraft (Pfeil 350 in Fig. 10) erfolgen.

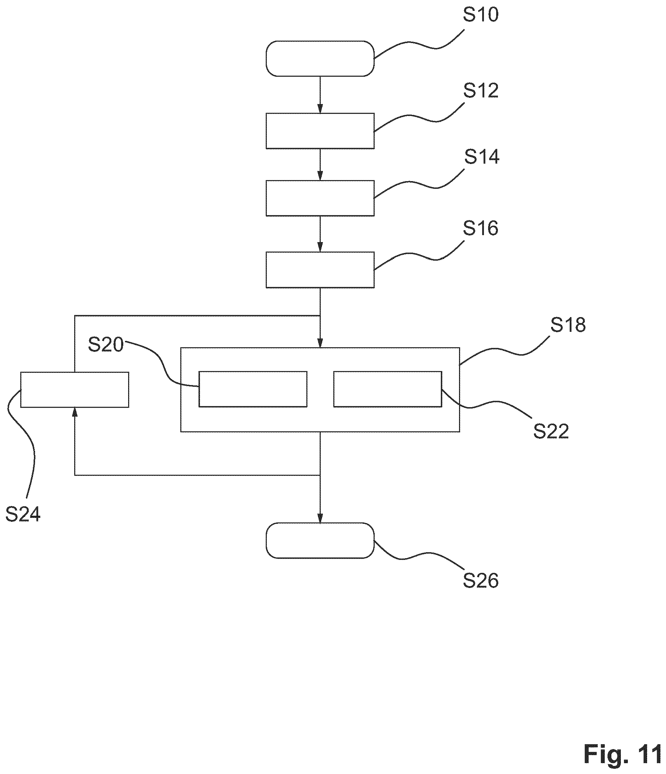

Mit Bezugnahme auf Fig. 11 wird anhand eines schematischen Blockdiagramm seine beispielhafte Ausgestaltung eines Verfahrens zur Herstellung eines Führungsrohres für ein medizinisches Instrument veranschaulicht.

Das Verfahren beginnt im Ausführungsbeispiel bei einem Schritt S10 und endet bei einem Schritt S26.

Ein Schritt S12 bezieht sich auf die Bereitstellung eines Ultrakurzpulslasers. Der Ultrakurzpulslaser kann Bestandteil einer geeigneten Fertigungsanlage zur Erzeugung des Führungsrohres sein. Ein sich anschließender Schritt S14 bezieht sich auf die Bereitstellung eines rohrförmigen Werkstücks, in dem mittels Ultrakurzpulslasern mehrere Aussparungen erzeugt werden sollen. Das Werkstück ist insbesondere ein durchmesserkleines Werkstück mit geringer Wandstärke.

In einem sich anschließenden Schritt S16 wird das Werkstück in einem Werkstückhalter aufgenommen und eingespannt. Dann kann die Bearbeitung zur Erzeugung einer Aussparung beginnen, vergleiche den Schritt S18. Die Bearbeitung erfolgt bei aktiviertem Ultrakurzpulslaser. Die Bearbeitung umfasst eine kombinierte Relativbewegung, umfassend eine translatorische Bewegung S20 und eine Schwenkbewegung S22. Auf diese Weise können Aussparungen im rohrförmigen Werkstück erzeugt werden, die etwa als Langloch oder Oval gestaltet sind, wobei die jeweilige Längserstreckung an die Längserstreckung des Werkstücks angepasst ist. Der Ultrakurzpulslaser kann durch die koordinierte Relativbewegung relativ zum Werkstück entlang eines geschlossenen Pfades bewegt werden, um einen Ausschnitt zu erzeugen.

Sofern weitere Aussparungen erzeugt werden sollen, erfolgt in einem weiteren Schritt S24 bei deaktiviertem Ultrakurzpulslaser eine Vorschubbewegung zwischen dem Werkstück und dem Ultrakurzpulslaser, damit anschließend der Schritt S18 erneut durchlaufen werden kann, um beabstandet von der zuvor erzeugten Aussparung eine weitere Aussparung zu erzeugen.

Die Schritte S18 bis S24 werden so lange durchlaufen, bis eine Reihe von Aussparungen entlang der Längserstreckung des Werkstücks erzeugt ist. Auf diese Weise lässt sich ein Führungsrohr mit einer Mehrzahl seitlicher Schlitze erzeugen, die in einer Reihe angeordnet sind.

Der Ultrakurzpulslaser kann filigrane Konturen im Werkstück erzeugen, indem entsprechende Ausschnitte freigeschnitten und entfernt werden. Demgemäß ist es auch vorstellbar, mehrere Reihen von Aussparungen zu erzeugen, wobei innerhalb einer Reihe Aussparungen relativ zueinander entlang der Längserstreckung versetzt sind, und wobei die Reihen am Umfang des Werkstücks relativ zueinander versetzt sind. Auch ist es grundsätzlich vorstellbar, Aussparungen mit unterschiedlichen Konturen miteinander zu kombinieren.

## Patentansprüche

1. Medizinisches Instrument (30, 230), insbesondere Arbeitseinsatz (32) für ein Zystoskop, Hysteroskop oder Resektoskop, das Folgendes aufweist:
- einen Schaft (34), der sich zwischen einem distalen Ende (38) und einem proximalen Ende (40) erstreckt,
- ein Gehäuse (36) beim proximalen Ende (40) des Schafts (34),
- zumindest einen Effektor (60) beim distalen Ende (38) des Schafts (34),
- zumindest ein Steuerelement (72) zur mechanischen Steuerung des Effektors (60),
wobei sich das Steuerelement (72) zumindest abschnittsweise durch ein sich parallel zum Schaft (34) erstreckendes Führungsrohr (70) erstreckt, und
wobei das Führungsrohr (70) zumindest abschnittsweise entlang seiner Längserstreckung (104) mit seitlichen Aussparungen (100) versehen ist, durch die das Führungsrohr (70) für Reinigungsmedien zugänglich ist.

2. Instrument (30, 230) nach Anspruch 1,
wobei der Effektor (60) ein auslenkbarer Hebel (62) am distalen Ende (38) des Schafts (34) ist, insbesondere ein Albarran-Hebel.

3. Instrument (30, 230) nach Anspruch 1 oder 2,
wobei am Gehäuse (36) ein Betätigungselement (74) zur Betätigung des Effektors (60) ausgebildet ist, mit dem das Steuerelement (72) nach distal oder proximal bewegbar ist.

4. Instrument (30, 230) nach einem der Ansprüche 1-3,
wobei das Führungsrohr (70) in einem ersten Umfangsabschnitt (120) am Schaft (34) befestigt ist, und
wobei die Aussparungen (100) auf einem davon abgewandten zweiten Umfangsabschnitt des Führungsrohres (70) ausgebildet sind.

5. Instrument (30, 230) nach einem der Ansprüche 1-4,
wobei mehrere Aussparungen (100) eine Reihe (110) entlang der Längserstreckung (104) des Führungsrohres (70) bilden.

6. Instrument (30, 230) nach einem der Ansprüche 1-5,
wobei die Aussparungen (100) als Langlöcher (136) oder Ellipsen gestaltet sind und eine Längserstreckung (138) sowie eine Querstreckung (140) aufweisen, und wobei die Längserstreckung (138) zumindest das Doppelte der Querstreckung (140) beträgt.

7. Instrument (30, 230) nach einem der Ansprüche 1-6,
wobei die Aussparungen (100) eine Längserstreckung (138) aufweisen, die zwischen 10 mm und 30 mm beträgt, insbesondere zwischen 15 mm und 25 mm, und/oder
wobei die Aussparungen (100) eine Querstreckung (140) aufweisen, die zwischen 0,15 mm und 1,0 mm beträgt, insbesondere zwischen 0,25 und 0,4 mm.

8. Instrument (30, 230) nach einem der Ansprüche 1-7,
wobei das Führungsrohr (70) einen Außendurchmesser (114) aufweist, der kleiner als 2,2 mm ist, vorzugsweise kleiner als 1,8 mm, weiter bevorzugt kleiner als 1,2 mm, und/oder
wobei das Führungsrohr (70) eine Wandstärke (116) aufweist, die kleiner 0,3 mm ist, vorzugsweise kleiner als 0,18 mm, weiter bevorzugt kleiner als 0,13 mm.

9. Instrument (30, 230) nach einem der Ansprüche 1-8,
wobei die Aussparungen (100) nachbearbeitungsarm oder nachbearbeitungsfrei mittels Ultrakurzpulslaserbearbeitung ausgeschnitten sind.

10. Instrument (30, 230) nach einem der Ansprüche 1-9,
wobei Kanten (126, 128) der Aussparungen (100), insbesondere Innenwandkanten (126), gratarm oder gratfrei gestaltet sind.

11. Instrument (30, 230) nach einem der Ansprüche 1-10,
wobei die Aussparungen (100) Seitenwände (124) aufweisen,
wobei einander gegenüberliegende Seitenwände (124) einer Aussparung (100) einen nach außen geöffneten Winkel (130) einschließen, der größer als 5° ist, vorzugsweise größer als 15°, weiter bevorzugt größer als 25°, und
wobei die Seitenwände (124) insbesondere radial zu einer Längsachse (112) des Führungsrohres (70) orientiert sind.

12. Instrument (30, 230) nach einem der Ansprüche 1-11,
wobei am Schaft (34) ein erstes Führungsrohr (70) und ein zweites Führungsrohr (70) angeordnet sind, die insbesondere außen am Schaft (34) befestigt sind,
wobei das erste Führungsrohr (70) ein erstes Steuerelement (72) und das zweite Führungsrohr (70) ein zweites Steuerelement (72) beherbergt, und
wobei das erste Steuerelement (72) und das zweite Steuerelement (72) beim distalen Ende (38) mit dem Effektor (60) gekoppelt sind.

13. Instrument (30, 230) nach einem der Ansprüche 1-12,
wobei der Schaft (34) mit dem Gehäuse (36) verbunden ist und insbesondere in das Gehäuse (36) mündet,
wobei das Gehäuse (36) proximal einen Zugang (90) in den Schaft (34) bereitstellt, und
wobei zumindest der Schaft (34) oder das Gehäuse (36) zumindest eine gegenüber einer Schaftachse (150) des Schafts (34) geneigte Bohrung (180, 182) aufweist, durch die ein von der Steuerung (162) des Effektors (60) genutzter Gehäuseraum (160) für Reinigungsmedien zugänglich ist.

14. Instrument (30, 230) nach Anspruch 13,
wobei das Führungsrohr (70) und der Schaft (34) über den Gehäuseraum (160) fluidisch miteinander verbunden sind.

15. Instrument (30, 230) nach Anspruch 13 oder 14,
wobei das Führungsrohr (70) in den Gehäuseraum (160) mündet.

16. Instrument (30, 230) nach einem der Ansprüche 13-15,
wobei zumindest eine geneigte Bohrung (180) als Querbohrung durch den Schaft (34) hin zum Gehäuseraum (160) ausgebildet ist, insbesondere als orthogonal zur Längsachse (150) des Schafts (34) orientierte Querbohrung.

17. Instrument (30, 230) nach einem der Ansprüche 13-16,
wobei zumindest eine geneigte Bohrung (182) als Verbindungsbohrung zwischen einem Gehäuseteil (152) beim proximalen Ende (40) des Schafts (34) und dem Gehäuseraum (160) ausgebildet ist, insbesondere unter einem spitzen Winkel zur Längsachse (150) des Schafts (34), der nach distal geöffnet ist.

18. Instrument (30, 230) nach einem der Ansprüche 1-17,
wobei das Steuerelement (72) an seinem proximalen Ende (246) mit einer Stange (240) gekoppelt ist, die sich durch ein Wandstück (242) in das Gehäuse (36) erstreckt, und
wobei ein proximales Ende (244) des Führungsrohres (70) vom Gehäuse (36) beabstandet ist.

19. Verfahren zur Herstellung eines Führungsrohres (70) eines medizinischen Instruments (30, 230), insbesondere eines Instruments (30, 230) nach einem der Ansprüche 1-18, mit den folgenden Schritten:
- Bereitstellung eines Werkstücks (306) in Form eines Halbzeugs mit einem dünnwandigen Rohrkörper (330) mit einer Längsachse (112), der sich zwischen einem ersten Ende (332) und einem zweiten Ende (334) erstreckt,
- Bereitstellung eines Ultrakurzpulslasers (310),
- Einspannen des Werkstücks (306) in einem Werkstückhalter (304),
- Betreiben des Ultrakurzpulslasers (310) zur Bearbeitung des Werkstücks (306) mittels Sublimation,
- Erzeugung einer Mehrzahl von Aussparungen (100) im Werkstück (306), umfassend:
- Erzeugen einer koordinierten Relativbewegung zwischen dem Rohrkörper (330) und dem Ultrakurzpulslaser (310) bei aktiviertem Ultrakurzpulslaser (310), umfassend eine translatorische Bewegung (322) entlang der Längsachse (112) und eine Schwenkbewegung (324) mit Bezug auf die Längsachse (112) zur Erzeugung eines Ausschnitts (340) im Werkstück (306), der eine Aussparung (100) definiert, und
- Erzeugen einer Vorschubbewegung zwischen dem Rohrkörper und dem Ultrakurzpulslaser (310) bei deaktiviertem Ultrakurzpulslaser (310), umfassend eine translatorische Bewegung (322) entlang der Längsachse (112), um mehrere Aussparungen (100) zu erzeugen, die voneinander beabstandet sind.

20. Verfahren nach Anspruch 19, ferner aufweisend:
- Abführen der Ausschnitte (340) radial nach außen, wobei die Ausschnitte (340) zueinander geneigte und zueinander gegenüberliegende Seitenwände (124) aufweisen, deren Neigungswinkel (130) in Richtung der Längsachse (110) zeigt, und/oder
- Rotieren des Werkstücks (306) um die Längsachse (112), bis die Ausschnitte (340) schwerkraftunterstützt abführbar sind.
